(19) Europäisches Patentamt
European Patent Office
Office européen des brevets

(11) **EP 2 514 449 A1**

(12) **EUROPEAN PATENT APPLICATION**
published in accordance with Art. 153(4) EPC

(43) Date of publication:
**24.10.2012 Bulletin 2012/43**

(51) Int Cl.:
***A61M 1/14*** *(2006.01)*

(21) Application number: **10837629.4**

(22) Date of filing: **15.12.2010**

(86) International application number:
**PCT/JP2010/072557**

(87) International publication number:
**WO 2011/074603 (23.06.2011 Gazette 2011/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.12.2009 JP 2009285912**

(71) Applicant: **Nipro Corporation
Osaka-shi, Osaka 531-8510 (JP)**

(72) Inventor: **UEDA, Mitsutaka
Osaka-shi
Osaka 531-8510 (JP)**

(74) Representative: **Bertsch, Florian Oliver et al
Kraus & Weisert
Patent- und Rechtsanwälte
Thomas-Wimmer-Ring 15
80539 München (DE)**

(54) **HEMODIALYSIS DEVICE**

(57) A hemodialyzer 1, a blood circuit 2, a blood pump 3 provided on the upstream side of the hemodialyzer 1 (an artery side blood circuit 21), a dialysis fluid supply line 4, and a draining line 6 are included and a dialysis condition calculating and displaying means 9 configured to, when a clearance of the hemodialyzer to be used is set and a blood flow rate is set, calculate and display a dialyzing fluid flow rate corresponding to the set clearance and the set blood flow rate is provided. The hemo-

dialysis apparatus is controlled by a control apparatus 8, and the dialysis condition calculating and displaying means 9 is electrically connected to the control apparatus 8. The dialysis condition calculating and displaying means 9 may be integrated in the control apparatus 8.

[Problem]

A hemodialysis apparatus which can calculate and display dialysis conditions with a simple operation while reducing costs is provided.

Fig.1

## Description

Technical Field

The present invention relates to a hemodialysis apparatus which can calculate and display dialysis conditions with a simple operation while reducing costs.

Background Art

The therapeutic dose in a hemodialysis therapy performed at one time is defined as an amount of dialysis. As indices of amount of dialysis include a urea extraction ratio and a Kt/V, and generally, the Kt/V is used.

The Kt/V is normally calculated by substituting serum urea concentrations at the time of start and end of the hemodialysis therapy, the amount of drainage, and the period of therapy into a predetermined arithmetic expression. Here, factors which affect the serum urea concentration at the end of the hemodialysis therapy include the amount of body fluid of a patient, the period of therapy, the amount of drainage, the overall mass transfer area coefficient (so-called "KoA") of the dialyzer to be used, the blood flow rate, and the dialyzing fluid flow rate. Normally, medical staff adjusts the blood flow rate from among these factors before starting the hemodialysis therapy to adjust the serum urea concentration at the end of the hemodialysis therapy, thereby adjusting the Kt/V.

However, the amount of drainage which is one of the factors which determine the serum urea concentration at the end of the hemodialysis therapy and also one of items which are substituted into the arithmetic expression for calculating the Kt/V is not necessarily the same between the hemodialysis therapy in the past and the hemodialysis therapy about to be performed.

The relationship between the blood flow rate and the Kt/V in the hemodialysis therapy differs by the patient and the dialyzer to be used. Therefore, even though the blood flow rate is adjusted, achievement of the Kt/V value as intended after having completed the hemodialysis therapy is difficult and, in addition, the operation for determining the blood flow rate required for achieving the intended Kt/V value demands enormous time and effort. Recently, there is an idea of reducing the amount of usage of the dialysis fluid to the extent possible in terms of cost.

Summary of Invention

Problems to be Solved by the Invention

In view of such circumstances described above, the present invention provides a hemodialysis apparatus which can calculate and display dialysis conditions with a simple operation while reducing costs.

Means for Solving the Problems

A hemodialysis apparatus according to the present invention includes a hemodialyzer, a blood circuit, a blood pump provided on the upstream side of the hemodialyzer, a dialysis fluid supply line, and a draining line, and

is provided with a dialysis condition calculating and displaying means configured to, when a clearance of the hemodialyzer to be used is set and the blood flow rate is set, calculate and display a dialyzing fluid flow rate corresponding to the set clearance and the set blood flow rate.

Here, when changing the blood flow rate, the dialysis condition calculating and displaying means may be configured to calculate and display new dialyzing fluid flow rates continuously corresponding to the set clearance and the changed blood flow rate in association with the change of the blood flow rate.

The dialysis condition calculating and displaying means may be integrated in a control apparatus described later. Although the present invention is generally described thus far, further understanding will be achieved by referring to several specific examples. These examples are provided in the specification only as exemplification, and do not limit the invention unless otherwise specified.

Advantages of the Invention

According to the present invention, the following advantage as described below is expected. In other words, once the clearance of the hemodialyzer to be used and the blood flow rate are set, the dialyzing fluid flow rate is calculated corresponding to the set clearance and the set blood flow rate. Also, by configuring the apparatus such that the dialyzing fluid flow rate is changed by changing the set blood flow rate, the dialyzing fluid flow rate can be lowered to the most efficient level within a settable range, and hence the setting operation of the efficient dialyzing fluid flow rate is achieved easily, and the cost of the dialysis therapy is reduced in terms of the amount of usage of the dialyzing fluid, so that the economical load of the patient can be alleviated.

Brief Description of the Drawings

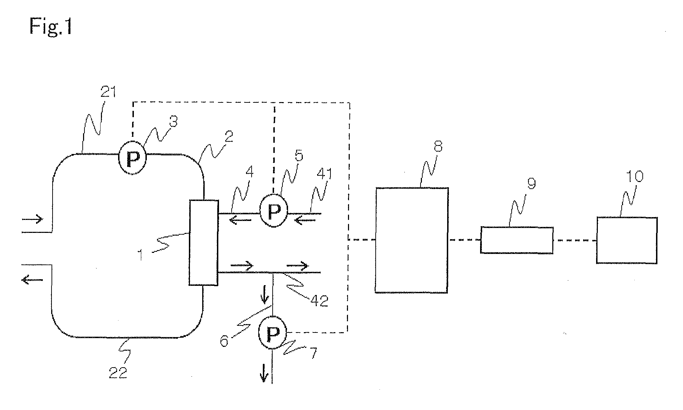

[Fig. 1] Fig. 1 is a schematic explanatory drawing showing a hemodialysis apparatus according to the present invention.

Best Modes for Carrying Out the Invention

A hemodialyzer, a blood circuit, a blood pump provided on the upstream side of the hemodialyzer, a dialysis fluid line, and a draining line are included, and a dialysis condition calculating and displaying means configured to, when a clearance of the hemodialyzer to be used is set and a blood flow rate is set, calculate and display a dialyzing fluid flow rate corresponding to the set clearance and the set blood flow rate is provided. When changing the blood flow rate, the dialysis condition calculating and displaying means is configured to calculate and display new dialyzing fluid flow rates continuously corresponding to the set clearance and the changed blood flow rate in association with the change of the blood flow rate.

Example 1

First of all, referring to the drawing, Example 1 will be described.
Fig. 1 is a schematic explanatory drawing showing a hemodialysis apparatus according to Example 1 according to the present invention.
The hemodialysis apparatus according to the present invention includes a hemodialyzer 1, a blood circuit 2, a blood pump 3 provided on the upstream side (an artery side blood circuit 21) of the hemodialyzer 1, a dialysis fluid supply line 4, and a draining line 6, and is provided with a dialysis condition calculating and displaying means 9 configured to, when a clearance of the hemodialyzer to be used is set and a blood flow rate is set, calculate and display a dialyzing fluid flow rate corresponding to the set clearance and the set blood flow rate as shown in Fig 1. The hemodialysis apparatus is controlled by a control apparatus 8, and the dialysis condition calculating and displaying means 9 is electrically connected to the control apparatus 8. The dialysis condition calculating and displaying means 9 may be integrated in the control apparatus 8. In the drawing, reference numeral 22 designates a vein side blood circuit, reference numeral 41 designates a dialyzing fluid supply line, reference numeral 42 designates a dialyzing fluid discharge line, reference numeral 5 designates a dialyzing fluid pump, reference numeral 7 designates a draining pump, and reference numeral 10 designates an input means with respect to the dialysis condition calculating and displaying means 9.
The dialysis condition calculating and displaying means 9 calculate and display the dialyzing fluid flow rate corresponding to the set clearance and the set blood flow rate when a clearance of the hemodialyzer to be used is set and the blood flow rate is set.
The dialyzing fluid pump 5 has a range of optimum amount of discharge and, as a result, the dialyzing fluid flow rate has a settable range, and the dialyzing fluid flow rate can only be set within the range. However, the dialyzing fluid flow rate under the given clearance and the given blood flow rate may fall out of the settable range of the dialyzing fluid flow rate. In such a case, it is required to change the blood flow rate and recalculate the dialyzing fluid flow rate within the settable range. Then, the determination of a new blood flow rate is generally has little choice but to be performed by trial and error. In order to solve such a problem, the hemodialysis apparatus according to this example is configured to continuously calculate and display the dialyzing fluid flow rate corresponding to the set clearance and the changed blood flow rate in keeping with the change of the blood flow rate. Therefore, with the hemodialysis apparatus in this example, the determination of the blood flow rate so that the dialyzing fluid flow rate corresponding to the set clearance and the changed blood flow rate falls within the settable range of the dialyzing fluid flow rate by trial and error is no longer necessary. In other words, the hemodialysis apparatus in this example has an advantage that the blood flow rate which allows the obtained dialyzing fluid flow rate to fall within the settable range can be determined by viewing a display panel.
Once the efficient dialyzing fluid flow rate is determined, the hemodialysis therapy is started at the set clearance and blood flow rate, and the dialyzing fluid flow rate by pressing a dialysis start button (not shown).
Incidentally, the clearance of the hemodialyzer varies from one hemodialyzer to another, and generally, the value at a blood flow rate of 200 ml and a dialyzing fluid flow rate of 500 ml is displayed. Then, when a large amount of blood filtration is required as in hemodiafiltration or in the case of a hemodialyzer which internally performs a large amount of blood filtration using a dialysis membrane having high water permeability, a clearance (CL) which indicates a removal performance of the hemodialyzer is determined by three values; namely, the blood flow rate (QB), the dialyzing fluid flow rate (QB) and the overall mass transfer area coefficient (KOA) except for a case where the dialyzing fluid flow rate or the blood flow rate is low when being used for an acute blood purification. The expression of relationship among CL, QB, QD, and KOA is referred to as a performance evaluation expression of the hemodialyzer, and is expressed as shown below.

$$\text{Expression 1}$$

$$CL = \frac{1 - e^{\alpha}}{\dfrac{1}{Q_D} - \dfrac{e^{\alpha}}{Q_B}}$$

where

$$e^{\alpha} = exp\left(\frac{KoA}{Q_B} - \frac{KoA}{Q_D}\right)$$

The dialysis condition calculating and displaying means 9 calculates the dialyzing fluid flow rate from the set clearance, the overall mass transfer area coefficient, and the blood flow rate using the expression 1 and displays the same. Here, normally, urea clearance is used as the set clearance.

However, since the KOA is originally an index which describes a phenomenon of only diffusion without being accompanied by the blood filtration, if the KOA is calculated from data measured under conditions in which the original definition cannot be satisfied as in the case of involving a large amount of blood filtration like hemodiafiltration or a case of the hemodialyzer which internally performs a large amount of blood filtration using the dialysis membrane having high water permeability, the obtained result has no original significance. Also, although there is a case where the KOA cannot be recognized as being constant as in the case where the dialyzing fluid flow rate or the blood flow rate is low when being used in the acute blood purification, the dialyzing apparatus in the present invention cannot be applied in such a case, and hence it is necessary to employ an operating method of the related art with the dialysis condition calculating and displaying means 9 is in an OFF state.

Reference Numerals

| | |
|---|---|
| 1 | hemodialyzer |
| 2 | blood circuit |
| 21 | artery side blood circuit |
| 22 | vein side blood circuit |
| 3 | blood pump |
| 4 | dialyzing fluid feed line |
| 41 | dialyzing fluid supply line |
| 42 | dialyzing fluid discharge line |
| 5 | dialyzing fluid pump |
| 6 | draining line |
| 7 | draining pump |
| 8 | control apparatus |
| 9 | dialysis condition calculating and displaying means |
| 10 | input means |

**Claims**

**1.** A hemodialysis apparatus including a hemodialyzer, a blood circuit, a blood pump provided on the upstream side of the hemodialyzer, a dialysis fluid supply line, and a draining line, comprising:

a dialysis condition calculating and displaying means configured to, when a clearance of the hemodialyzer to be used is set and a blood flow rate is set, calculate and display a dialyzing fluid flow rate corresponding to the set clearance and the set blood flow rate.

**2.** The hemodialysis apparatus according to Claim 1, wherein when changing the blood flow rate, the dialysis condition calculating and displaying means is configured to calculate and display new dialyzing fluid flow rates continuously corresponding to the set clearance and the changed blood flow rate in association with the change of the blood flow rate.

Fig.1

| | INTERNATIONAL SEARCH REPORT | International application No. |
|---|---|---|
| | | PCT/JP2010/072557 |

**A. CLASSIFICATION OF SUBJECT MATTER**
*A61M1/14*(2006.01)i

According to International Patent Classification (IPC) or to both national classification and IPC

**B. FIELDS SEARCHED**

Minimum documentation searched (classification system followed by classification symbols)
A61M1/14

Documentation searched other than minimum documentation to the extent that such documents are included in the fields searched
Jitsuyo Shinan Koho        1922–1996    Jitsuyo Shinan Toroku Koho    1996–2011
Kokai Jitsuyo Shinan Koho    1971–2011    Toroku Jitsuyo Shinan Koho    1994–2011

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2006-285488 A (Asahi Kasei Medical Co., Ltd.), 19 October 2006 (19.10.2006), entire text; all drawings (Family: none) | 1-2 |
| A | WO 1995/032010 A1 (BAXTER INTERNATIONAL INC.), 30 November 1995 (30.11.1995), entire text; all drawings & US 5507723 A       & EP 0711182 B1 & DE 69531137 T2      & AU 698652 B & AU 2386695 A       & AT 243539 T | 1-2 |
| A | JP 2007-029705 A (JMS Co., Ltd.), 08 February 2007 (08.02.2007), entire text; all drawings (Family: none) | 1-2 |

| ☒ Further documents are listed in the continuation of Box C. | ☐ See patent family annex. |
|---|---|

| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
|---|---|

| Date of the actual completion of the international search<br>08 March, 2011 (08.03.11) | Date of mailing of the international search report<br>15 March, 2011 (15.03.11) |
|---|---|
| Name and mailing address of the ISA/<br>Japanese Patent Office | Authorized officer |
| Facsimile No. | Telephone No. |

Form PCT/ISA/210 (second sheet) (July 2009)

**EP 2 514 449 A1**

**INTERNATIONAL SEARCH REPORT**

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2005-245667 A  (Koninklijke Philips Electronics N.V.), 15 September 2005 (15.09.2005), entire text; all drawings & WO 2005/086064 A2     & CN 1930571 A | 1-2 |

Form PCT/ISA/210 (continuation of second sheet) (July 2009)